# EUROPEAN PATENT APPLICATION

(11) **EP 3 216 873 A1**
(43) Date of publication of application: **13.09.2017**
(21) Application number: 16159205.0
(22) Date of filing: 08.03.2016
(51) Int. Cl.: C12Q 1/68, G06F 19/22

(54) **COMBINATION OF STRUCTURAL VARIATIONS AND SINGLE NUCLEOTIDE CHANGES IN ONE STATISTICAL MODEL FOR IMPROVED THERAPY SELECTION**

(71) Applicant: Curetis GmbH, 71088 Holzgerlingen (DE)
(72) Inventor: BACKES, Christina, 66125 Saarbrücken (DE); GALATA, Valentina, 66123 Saarbrücken (DE); KELLER, Andreas, 66346 Püttlingen (DE); SCHMOLKE, Susanne, 91052 Erlangen (DE); STÄHLER, Friedrich, 69493 Hirschberg an der Bergstrasse (DE)
(74) Representative: Schnappauf, Georg

(57) **Abstract**

The invention relates to a method of determining an antimicrobial drug resistance profile for a microorganism, wherein nucleic acid sequences of the microorganism are analyzed for structural variations of the genome comprising at least a change in the genome comprising more than one base, as well as for single nucleotide polymorphisms (SNPs), as well as a method of determining an infection of a patient with a microorganism potentially resistant to antimicrobial drug treatment and a method of selecting a treatment of a patient suffering from an infection with a potentially resistant microorganism, wherein the data of the antimicrobial drug resistance profile are applied.

## Description

The present invention relates to a method of determining an antimicrobial drug resistance profile for a microorganism, wherein nucleic acid sequences of the microorganism are analyzed for structural variations of the genome comprising at least a change in the genome comprising more than one base, as well as for single nucleotide polymorphisms (SNPs), respectively single nucleotide variants, as well as a, e.g. diagnostic, method of determining an infection of a patient with a microorganism potentially resistant to antimicrobial drug treatment and a method of selecting a treatment of a patient suffering from an infection with a potentially resistant microorganism, wherein the data of the antimicrobial drug resistance profile are applied.

Antibiotic resistance is a form of drug resistance whereby a sub-population of a microorganism, e.g. a strain of a bacterial species, can survive and multiply despite exposure to an antibiotic drug. It is a serious health concern for the individual patient as well as a major public health issue. Timely treatment of a bacterial infection requires the analysis of clinical isolates obtained from patients with regard to antibiotic resistance, in order to select an efficacious therapy. Generally, for this purpose an association of the identified resistance with a certain microorganism (i.e. ID) is necessary.

Antibacterial drug resistance (ADR) represents a major health burden. According to the World Health Organization's antimicrobial resistance global report on surveillance, ADR leads to 25,000 deaths per year in Europe and 23,000 deaths per year in the US. In Europe, 2.5 million extra hospital days lead to societal cost of 1.5 billion euro. In the US, the direct cost of 2 million illnesses leads to 20 billion dollar direct cost. The overall cost is estimated to be substantially higher, reducing the gross domestic product (GDP) by up to 1.6%.

In general the mechanisms for resistance of bacteria against antimicrobial treatments rely to a very substantial part on the organism's genetics. The respective genes or molecular mechanisms are either encoded in the genome of the bacteria or on plasmids that can be interchanged between different bacteria. The most common resistance mechanisms include:
1) Efflux pumps are high-affinity reverse transport systems located in the membrane that transports the antibiotic out of the cell, e.g. resistance to tetracycline.
2) Specific enzymes modify the antibiotic in a way that it loses its activity. In the case of streptomycin, the antibiotic is chemically modified so that it will no longer bind to the ribosome to block protein synthesis.
3) An enzyme is produced that degrades the antibiotic, thereby inactivating it. For example, the penicillinases are a group of beta-lactamase enzymes that cleave the beta lactam ring of the penicillin molecule.

In addition, some pathogens show natural resistance against drugs. For example, an organism can lack a transport system for an antibiotic or the target of the antibiotic molecule is not present in the organism.

Pathogens that are in principle susceptible to drugs can become resistant by modification of existing genetic material (e.g. spontaneous mutations for antibiotic resistance, happening in a frequency of one in about 100 mio bacteria in an infection) or the acquisition of new genetic material from another source. One example is horizontal gene transfer, a process where genetic material contained in small packets of DNA can be transferred between individual bacteria of the same species or even between different species. Horizontal gene transfer may happen by transduction, transformation or conjugation. Usually, the expression of resistance imparting markers is induced only by presence of a drug.

Generally, testing for susceptibility/resistance to antimicrobial agents is performed by culturing organisms in different concentrations of these agents.

In brief, agar plates are inoculated with patient sample (e.g. urine, sputum, blood, stool) overnight. On the next day individual colonies are used for identification of organisms, either by culturing or using mass spectroscopy. Based on the identity of organisms new plates containing increasing concentration of drugs used for the treatment of these organisms are inoculated and grown for additional 12 - 24 hours. The lowest drug concentration which inhibits growth (minimal inhibitory concentration - MIC) is used to determine susceptibility/resistance for tested drugs. The process takes at least 2 to 3 working days during which the patient is treated empirically. Automated systems exist from several companies, e.g. Biomeriux (Vitek), Beckman Coulter (Microscan). A significant reduction of time-to-result is needed especially in patients with life-threatening disease and to overcome the widespread misuse of antibiotics.

Recent developments include PCR based test kits for fast bacterial identification (e.g. Biomerieux Biofire Tests, Curetis Unyvero Tests). With these test the detection of selected resistance loci is possible for a very limited number of drugs, but no correlation to culture based AST is given. Mass spectroscopy is increasingly used for identification of pathogens in clinical samples (e.g. Bruker Biotyper), and research is ongoing to establish methods for the detection of susceptibility/resistance against antibiotics.

The use of molecular techniques for direct detection of MRSA has become more commonplace especially for screening purposes. Resistance to methicillin is mediated via the mec operon which is part of the staphylococcal cassette chromosome mec (SCCmec). Recently PCR tests were introduced that are based on the detection of the right extremity sequence of the SCCmec in combination with S. aureus specific marker. Initial reports exist that describe culture based susceptibility reports despite detection of the presence of a resistance conferring gene.

For some drugs such it is known that at least two targets are addressed, e.g. in case of Ciprofloxacin (drug bank ID 00537; http://www.drugbank.ca/drugs/DB00537) targets include DNA Topoisomerase IV, DNA Topoisomerase II and DNA Gyrase. It can be expected that this is also the case for other drugs although the respective secondary targets have not been identified yet. In case of a common regulation, both relevant genetic sites would naturally show a co-correlation or redundancy.

It is known that drug resistance can be associated with genetic modifications such as polymorphisms or gene duplications/deletions. This holds for viruses, where resistance testing is established clinical practice (e.g. HIV genotyping). More recently, it has been shown that resistance has also genetic causes in bacteria and even higher organisms, such as humans where tumors resistance against certain cytostatic agents can be linked to genomic mutations.

Wozniak et al. (BMC Genomics 2012, 13(Suppl 7):S23) disclose genetic determinants of drug resistance in Staphylococcus aureus based on genotype and phenotype data. Stoesser et al. disclose prediction of antimicrobial susceptibilities for Escherichia coli and Klebsiella pneumoniae isolates using whole genomic sequence data (J Antimicrob Chemother 2013; 68: 2234-2244).

Chewapreecha et al (Chewapreecha et al (2014) Comprehensive Identification of single nucleotid polymorphisms associated with beta-lactam resistance within pneumococcal mosaic genes. PLoS Genet 10(8): e1004547) used a comparable approach to identify mutations in gram-positive Streptococcus Pneumonia.

In recent studies, genetic tests are taken into account that consider variations in the genome of a microorganism, e.g. a bacterial microorganism. In previous works it could be shown that a faster decision for a treatment could be made using changes in single bases. However, this does not necessarily apply to all antimicrobial drugs, e.g. antibiotics, tested.

For genetic diagnosis and prognosis in human pathologies (including diagnosis, predicting response to therapies of humans, prediction of drug response of bacteria and viruses and many similar tasks) different "scales" can be considered.

First, it is possible to consider structural variations in genomes. This includes inclusion of repetitive elements, copy number variations (gains and losses of single genes or larger parts of chromosomes), gene fusions, translocations, inclusion/addition of new genes, and other more rare events. Respective diagnostic means are used e.g. for fragile X syndrome or in different cancers where gene amplification is known to be closely related to the prognosis of patients. As an example of a structural variation, an efflux pump can be present on a plasmid additionally in a genome. Such efflux pump then can transport a medicine/drug like an antibiotic out of the organism, so that it cannot be effective. Thus, a bacterium having such efflux pump on a plasmid is resistant. Secondly, beyond the structural variations we can on a higher resolution scale interpret single nucleotide variations. These can include besides the actual exchange of a single base in the genetic code also the gain or loss of one base in the genetic code (denoted as small insertion or deletion). Respective single nucleotide variants are used in many research areas such as oncology for predicting the effectiveness of therapies. Among the most popular examples are mutations in the gene KRAS.

Besides oncology, neurology and cardiology, genetic testing is increasingly applied to infectious diseases. Here, the genetic code of the pathogenic organism can be investigated instead or in addition to the genetic code of the host. While therapies for the human immunodeficiency virus are already frequently determined using sequencing of the virus, the field for the more complex bacteria is currently emerging. The question is now whether for pathogens the first or second variant, i.e. structural variations or single nucleotide polymorphisms, leads to more accurate therapy predictions. This is especially important since for antimicrobial treatment a very large number of different therapies exist that belong to various drug classes with different modes of action.

The fast and accurate detection of infections with microorganisms, particularly microbial species, and the prediction of response to anti-microbial therapy represent still a high unmet clinical need.

### Summary of the invention

The inventors found out that a combination of structural variations in the genome that relate to more than one base, particularly at least one gene or more genes in an open reading frame, with single nucleotide polymorphisms (SNPs) can improve the diagnosis of resistant / susceptible microorganisms, particularly bacterial microorganisms, to antimicrobial, e.g. antibiotic, drugs.

According to a first aspect the present invention relates to a method of determining an antimicrobial drug, e.g. antibiotic, resistance, respectively susceptibility, profile for a microorganism, particularly a bacterial microorganism, comprising:
obtaining or providing a first data set of nucleic acid sequences of a plurality of clinical isolates of the microorganism, wherein optionally at least a part of the nucleic acid sequences of the first data set are assembled; and/or obtaining or providing a first data set of nucleic acid sequences of a plurality of clinical isolates of the microorganism and aligning the nucleic acid sequences of the first data set to at least one, preferably one, reference sequence, e.g. also a pan genome;
analyzing the nucleic acid sequences of the first data set for structural variations of the genome comprising at least a change in the genome comprising more than one base, and analyzing the nucleic acid sequences of the first data set for single nucleotide polymorphisms (SNPs) to obtain a third data set of structural variants;
providing a second data set of antimicrobial drug, e.g. antibiotic, resistance and/or susceptibility of the plurality of clinical isolates of the microorganism;
correlating the third data set with the second data set and statistically analyzing the correlation; and
determining the structural variations in the genome of the microorganism associated with antimicrobial drug, e.g. antibiotic, resistance.

Furthermore discloses is - in a second aspect - a, e.g. diagnostic, method of determining an infection of a patient with a microorganism, particularly a bacterial microorganism potentially resistant to antimicrobial drug treatment, comprising the steps of:
a) obtaining or providing a sample containing or suspected of containing a microorganism, particularly a bacterial microorganism, from the patient;
b) determining the presence of at least one structural variation of the genome comprising at least a change in the genome comprising more than one base, and at least one single nucleotide polymorphism (SNP) in at least one genetic sequence of the microorganism, particularly bacterial microorganism, as determined by the method of the first aspect, wherein the presence of said at least one structural variation and said single nucleotide polymorphism is indicative of an infection with an antimicrobial drug resistant microorganism in said patient.

In addition, a method of selecting a treatment of a patient suffering from an infection with a potentially resistant microorganism, particularly bacterial microorganism is disclosed in a third aspect, comprising the steps of:
a) obtaining or providing a sample containing or suspected of containing a microorganism, particularly a bacterial microorganism, from the patient;
b) determining the presence of at least one structural variation of the genome comprising at least a change in the genome comprising more than one base, and at least one single nucleotide polymorphism (SNP) in at least one genetic sequence of the microorganism, particularly bacterial microorganism, as determined by the method of the first aspect, wherein the presence of said at least one structural variation is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism, particularly the bacterial microorganism.

In a further aspect the present invention is directed to a computer program product comprising computer executable instructions which, when executed, perform a method according to either of the first, second and third aspect.

Even further aspects and embodiments of the invention are disclosed in the dependent claims and can be taken from the following description and examples, without being limited thereto.

### Detailed description of the invention

### Definitions

Unless defined otherwise, technical and scientific terms used herein have the same meaning as commonly understood by one of ordinary skill in the art to which this invention belongs.

Susceptibility herein means that isolates are inhibited by a certain concentration of an antimicrobial agent, whereas resistance means that isolates are not inhibited

An "antimicrobial drug" in the present invention refers to a group of drugs that includes antibiotics, antifungals, antiprotozoals, and antivirals. According to certain embodiments, the antimicrobial drug is an antibiotic.

The term "nucleic acid molecule" refers to a macromolecule comprising nucleotides, particularly a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA.

The term "nucleic acid sequence information" relates to information which can be derived from the sequence of a nucleic acid molecule, i.e. the nucleic acid sequence, such as the sequence itself or a variation in the sequence as compared to a reference sequence. A genetic sequence can thereby encompass coding as well as non-coding parts.

The term "mutation" relates to a variation in the sequence as compared to a reference sequence. Such a reference sequence can be e.g. determined in a predominant wild type organism or another reference organism, e.g. a defined and known bacterial strain or substrain. A mutation is for example a deletion of one or multiple nucleotides, an insertion of one or multiple nucleotides, or substitution of one or multiple nucleotides, duplication of one or a sequence of multiple nucleotides, translocation of one or a sequence of multiple nucleotides, e.g. also a single nucleotide polymorphism (SNP). The term "single nucleotide polymorphism" (SNP) is thereby synonymous to the term "single nucleotide variant" (SNV), and both refer to the same.

In the context of the present invention a "sample" is a sample which comprises at least one nucleic acid molecule from a bacterial microorganism. Examples for samples are: cells, tissue, biopsy specimens, body fluids such as blood, urine, saliva, sputum, plasma, serum, cell culture supernatant, swab sample and others. According to certain embodiments, the sample is a patient sample (clinical isolate).

New and highly efficient methods of sequencing nucleic acids referred to as next generation sequencing have opened the possibility of large scale genomic analysis. The term "next generation sequencing" or "high throughput sequencing" refers to methods achieving a higher throughput in sequencing, e.g. high-throughput sequencing technologies that parallelize the sequencing process, producing thousands or millions of sequences at once, or methods producing longer reads and are read out faster. Examples include Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing, Sequencing By Hybridization, Amplicon Sequencing, GnuBio.

Within the present description the term "microorganism" comprises the term microbe. The type of microorganism is not particularly restricted, unless noted otherwise or obvious, and, for example, comprises bacteria, viruses, fungi, microscopic algae und protozoa, as well as combinations thereof. According to certain aspects, it refers to one or more bacterial species, being either Gram-negative or Gram-positive, e.g. one or more of Acinetobacter, Escherichia, e.g. E.coli, Enterobacter, Klebsiella, Proteus, Pseudomonas, Salmonella, Serratia, Shigella and/or Staphylococcus species.

A reference to a microorganism or microorganisms in the present description comprises a reference to one microorganism as well a plurality of microorganisms, e.g. two, three, four, five, six or more microorganisms.

A vertebrate within the present invention refers to animals having a vertebrae, which includes mammals - including humans, birds, reptiles, amphibians and fishes. The present invention thus is not only suitable for human medicine, but also for veterinary medicine.

According to certain embodiments, the patient in the present methods is a vertebrate, more preferably a mammal and most preferred a human patient.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

Regarding the dosage of the antimicrobial, e.g. antibiotic, drugs, it is referred to the established principles of pharmacology in human and veterinary medicine. For example, Forth, Henschler, Rummel "Allgemeine und spezielle Pharmakologie und Toxikologie", 9th edition, 2005 might be used as a guideline. Regarding the formulation of a ready-to-use medicament, reference is made to "Remington, The Science and Practice of Pharmacy", 22nd edition, 2013.

Assembling of a nucleic acid, e.g. gene, sequence can be carried out by any known method and is not particularly limited.

According to certain embodiments, mutations that were found using alignments can also be compared or matched with alignment-free methods, e.g. for detecting single base exchanges, for example based on contigs that were found by assemblies. For example, reads obtained from sequencing can be assembled to contigs and the contigs can be compared to each other.

In the description, the term "structural variations" is used equivalently to the term "structural changes", and both refer to the same phenomenon within the scope of this invention.

A structural variation comprising a change in the genome comprising more than one base refers to a structural variation wherein at least two bases, preferably at least four bases, in a nucleic acid sequence of a genome of a microorganism that are adjacent are changed, and can refer to e.g. a deletion of multiple (2, e.g. 4, or more) nucleotides, an insertion of multiple (2, e.g. 4, or more) nucleotides, a substitution of multiple (2, e.g. 4, or more) nucleotides, a duplication of a sequence of multiple (2, e.g. 4, or more) nucleotides, or a translocation of a sequence of multiple (2, e.g. 4, or more) nucleotides. According to certain embodiments, a structural variation affects a sequence length of at least about 50 bases, preferably at least about 100 bases, further preferably at least about 1 Kb (= 1000 bases). According to certain embodiments, a structural variation affects a sequence length of at most 300 Mb (Mega base = 1000000 bases), e.g. of at most 30 Mb, e.g. of at most 3Mb. In case the term "structural variation refers to a change in the genome of 4 or more bases, e.g. at least about 50 bases, preferably at least about 100 bases, further preferably at least about 1 Kb, the term single nucleotide polymorphism can be understood to include also small indels (insertions or deletions) of up to at most 3 bases, e.g. up to two bases. According to certain embodiments, a structural variation can comprise bigger parts sections of the genome, e.g. at least one whole gene in the genome of the microorganism, or even more genes in an open reading frame. According to certain embodiments, structural variations refer to inclusion of repetitive elements, copy number variations (gains and losses of single genes or larger parts of chromosomes), gene fusions, translocations and other more rare events. According to certain embodiments, at least one inclusion of repetitive elements, one copy number variation (gains and losses of single genes or larger parts of chromosomes), one gene fusion, and/or translocation of single genes or larger parts of chromosomes is observed in the present methods as a structural variation.

A single nucleotide polymorphism (SNP) refers within the scope of the invention to a variation in a single nucleotide within a genome, which can result from e.g. an addition, deletion, substitution, insertion or translocation of a single nucleotide.

In the present invention, a reference sequence is not particularly limited, as long as it is useful as a reference for one or more unknown nucleic acid sequences in one or more samples. It can, for example, be one or more reference genomes, a pan genome or one or more centroids. A pan genome, also referred to as supra-genome, can describe the full complement of genes in a clade, e.g. a certain species in bacteria, which can vary among related strains. According to certain embodiments, the reference sequences comprise one or more centroids, wherein a centroid is a representative of a gene group/family/cluster of a genome, e.g. of a microorganism. Centroids can be for example extracted from the database MetaRef (http://metaref.org/), which was used in the present examples, with the extraction from the data base being carried out particularly on November 24, 2014. After the extraction the data from the MetaRef database can be updated continually for further experiments. A list of centroids can be extracted for each organism separately or as a whole. The centroid information, e.g. for annotation, can be extracted from databases like IMG (http://img.jgi.doe.gov/), as in the present case, or NCBI. According to certain embodiments, alignment is carried out using a pan genome.

According to a first aspect, the present invention relates to a method of determining an antimicrobial drug, e.g. antibiotic, resistance/susceptibility profile for a microorganism, particularly a bacterial microorganism, comprising: obtaining or providing a first data set of nucleic acid, e.g. gene, sequences of a plurality of clinical isolates of the microorganism, wherein optionally at least a part of the nucleic acid, e.g. gene, sequences of the first data set are assembled; and/or obtaining or providing a first data set of nucleic acid, e.g. gene, sequences of a plurality of clinical isolates of the microorganism and aligning the nucleic acid, e.g. gene, sequences of the first data set to at least one, preferably one, reference sequence;
analyzing the nucleic acid, e.g. gene, sequences of the first data set for structural variations of the genome comprising at least a change in the genome comprising more than one base, and analyzing the nucleic acid, e.g. gene, sequences of the first data set for single nucleotide polymorphisms (SNPs) to obtain a third data set of structural variants;
providing a second data set of antimicrobial drug, e.g. antibiotic, resistance and/or susceptibility of the plurality of clinical isolates of the microorganism;
correlating the third data set with the second data set and statistically analyzing the correlation; and
determining the structural variations in the genome of the microorganism associated with antimicrobial drug, e.g. antibiotic, resistance.

In this method, as well as the other methods of the invention, the first data set of nucleic acid, e.g. gene, sequences of a plurality of clinical isolates can be provided or obtained in any way, preferably non-invasive, and can be e.g. provided from *in vitro* samples.

According to certain embodiments, the obtaining or providing of nucleic acid, e.g. gene, sequences of a plurality of clinical isolates in this method - as well as the other methods of the invention - can comprise the following:
A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of at least one microorganism of interest, particularly a bacterial microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be used to identify the nucleic acids of the microorganism to be identified, by known methods, e.g. fingerprinting methods, comparing genomes and/or aligning to at least one, or more, reference sequences of one or more species of the microorganism of interest, e.g. a reference genome and/or centroids, etc., forming a third data set of, optionally aligned, nucleic acid sequences, e.g. genes, for a microorganism - discarding additional data from other sources, e.g. the vertebrate. For the present method, also the raw data can be used and/or assemblies, at least in part, can be used for forming the third data set. Thus, according to certain embodiments, at least a part of the nucleic acid, e.g. gene, sequences of the first data set can be assembled, wherein assembly can be carried out by any known method and is not particularly limited. In addition, also data from reference sequences, e.g. centroids and/or genomes of known species, e.g. from bacterial species that are already known, e.g. using databases like MetaRef - which can provide pan genomes - and/or at the NCBI, can be used in the first data set and/or for evaluation of the first data set.

For some organisms, it might be useful in genome-wide association studies to reference the points of interest, e.g. structural variations and/or SNPs, to one constant reference for enhanced standardization. In case of the human with a high consistency of the genome and 99% identical sequences among individuals this is easy and represents the standard, as corresponding reference genomes are available in databases.

In case of organisms that trigger infectious diseases (e.g. bacteria and viruses) this is much more difficult, though, and particularly also genetic variations like structural variations and/or SNPs that are not on genes, particularly known genes, can be missed when aligning sequence data to a reference genome. One possibility to overcome this is to fall back on a virtual pan-genome which contains all sequences of a certain genus or to perform reference free variation calling. A further possibility is the analysis of a huge amount of reference sequences, e.g. using MetaRef, and even all available references, which is much more complex. Therein all *n* references from a database (e.g. RefSeq) are extracted and compared with the newly sequenced bacterial genomes k. After this, matrices (% of mapped reads, % of covered genome) can be applied and the data can be compared to several reference sequences. In such a case, n x *k* complete alignments are carried out. Having a big number of references, stable results can be obtained.

In the present method, nucleic acid, e.g. gene, sequence of the first data set can also be assembled, at least in part, according to certain embodiments with known methods, e.g. by de-novo assembly or mapping assembly, reference guided assembly. The sequence assembly is not particularly limited, and any known genome assembler can be used, e.g. based on Sanger, 454, Solexa, Illumina, SOLid technologies, etc., as well as hybrids/mixtures thereof.

According to certain embodiments, the data of nucleic acids of different origin than the microorganism of interest, e.g. a bacterial microorganism, can be removed after the nucleic acids of interest are identified, e.g. by filtering the data out. Such data can e.g. include nucleic acids of a patient, e.g. the vertebrate, e.g. human, and/or other microorganisms, etc. This can be done by e.g. computational subtraction, as developed by Meyerson et al. 2002. For this, also aligning to the genome of the vertebrate, etc., is possible. For aligning, several alignment-tools are available. This way the original data amount from the sample can be drastically reduced.

After such removal of "excess" data, obtaining the third data set can be carried out for the microorganism, e.g. a bacterial microorganism, as described above.

Using these techniques, structural variations and SNPs in the genome, e.g. in the gene sequences, of the microorganism of interest, e.g. a bacterial microorganism, can be obtained for various species.

When testing these same species for antimicrobial drug, e.g. antibiotic, susceptibility of a number of antimicrobial drugs, e.g. antibiotics, e.g. using standard culturing methods on dishes with antimicrobial drug, e.g. antibiotic, intake, as e.g. described below, the results of these antimicrobial drug, e.g. antibiotic, susceptibility tests can then be cross-referenced/correlated with the structural variations in the genome of the respective microorganism. Using several, e.g. 50 or more than 50, 100 or more than 100, 200 or more than 200, 400 or more than 400, 800 or more than 800, 900 or more than 900, 1000 or more than 1000, or 1100 or more than 1100 different isolates of the same or different species of a microorganism, statistical analysis can be carried out on the obtained cross-referenced data between genetic variations and antimicrobial drug, e.g. antibiotic, susceptibility for these microorganisms, using known methods.

Regarding culturing methods, which are nor limited, samples of microorganisms can be e.g. cultured overnight. On the next day individual colonies can be used for identification of organisms, either by culturing or using mass spectroscopy. Based on the identity of organisms new plates containing increasing concentration of antibiotics used for the treatment of these organisms are inoculated and grown for additional 12 - 24 hours. The lowest drug concentration which inhibits growth (minimal inhibitory concentration - MIC) can be used to determine susceptibility/resistance for tested antibiotics.

Also, resistance testing can be carried out by determining e.g. known resistance genes in the different isolates, like in case of methicillin resistant Staphylococcus aureus (MRSA) and methicillin susceptible Staphylococcus aureus (MSSA). For determining resistances, respectively susceptibility, the data from culturing methods and/or from determining known resistance genes, as well as data obtained in different ways, e.g. based on mass spectrometry (possibly also in connection with culturing) can be used.

Correlation of the genetic variations with antimicrobial drug, e.g. antibiotic, resistance can be carried out in a usual way and is not particularly limited. For example, resistances can be correlated to structural variations and SNPs in the whole genome of the respective microorganism or only parts thereof, for example only coding parts of the genome. In some cases even only genetic variations, i.e. structural variations and SNPS in nucleic acid molecules with certain nucleic acid sequences, e.g. genes, e.g. certain genes, or certain mutations in nucleic acid molecules with certain nucleic acid sequences, e.g. genes, can be determined. After correlation, statistical analysis can be carried out.

According to certain embodiments, the data of the first data set, particularly structural variations and/or SNPs, can be filtered prior to a possible annotation to a pan-genome and/or reference genome(s) and the correlation with the resistance/susceptibility data, e.g. when determining structural variations.

For example, to reduce the number of similar annotations, e.g. for structural variations and/or SNPs, they can be filtered and aggregated by one or more of the following:
- Only annotations for which the considered structural variation and/or SNP lies on a protein can be kept and the further data discarded
- Only annotations which do not contain "hypothetical proteins" can be kept
- Annotations can be sorted by identification number (ID), e.g. for SNPs and/or structural variation, and nucleic acid sequence, e.g. gene product
- For a unique pair of IDs and nucleic acid sequences, e.g. gene products, only the first annotation can be kept, e.g. in case of multiple nucleic acid sequences, e.g. coding certain genes, in a genome

Also, according to certain embodiments, the following structural variations and/or SNPs can be excluded:
1. Constant features and phenotypes (same value or only NA (not applicable)) can be removed (e.g. centroids present in all samples or phenotypes with the result "resistant" for all samples)
2. Almost constant features and phenotypes can also be removed, e.g. features whose most frequent value was in >=95% of all samples, ignoring NA values, can be removed (e.g. a centroid is present in >=95% of all samples)
   ∘ Also phenotypes whose most frequent value was in >=90% of all samples, ignoring NA values, can be removed (e.g. >=90% of all samples are resistant)
3. In addition, only drugs with non-missing data for at least 10% of the samples can be kept.
4. Mutations, e.g. SNPs, without any annotation or mutations, e.g. SNPs, whose all annotations contain flag "synonymous", so that only mutations, e.g. SNPs, with at least one non-synonymous annotation, e.g. a non-synonymous coding, are considered

For statistical analysis, as in the examples, e.g. Fisher's exact two-sided test can be applied with subsequent p-value adjustment over all phenotypes together using e.g. familywise error rate (FWER) or FDR (false discovery rate) and p-value threshold of 0.01 (corresponding to 10⁻², respectively 1e-2). Additionally, 10 permutation tests can be performed by permuting each phenotype separately and applying Fisher's exact test, e.g. to the centroid presence matrix and permuted phenotypes. Regarding centroids, the results then can be further filtered by centroid annotation, i.e.
1. Centroids without a gene product name can optionally be removed
2. Centroids whose gene product name contains "putative", "predicted" or "hypothetical" can be removed
3. If there are centroids with same gene product name and gene symbol than only the first one can be kept
4. Centroids without GeneBank accession can be removed

According to certain embodiments, the structural variations and/or SNPs can be annotated to a pan-genome of the microorganism and/or annotated to one or more reference sequences, e.g. centroids, of the microorganism. The construction of a pan-genome is not particularly limited and can be done using known methods.

However, other suitable reference genomes (e.g. used in the Examples, but also for other microorganisms) can be found at publicly available data bases like at the NCBI or from MetaRef.

Statistical analysis of the correlation of the nucleic acid, e.g. gene, mutations with antimicrobial drug, e.g. antibiotic, resistance is not particularly limited and can be carried out, depending on e.g. the amount of data, in different ways, for example using analysis of variance (ANOVA), Student's t-test or Fisher's exact test, for example with a sample size n of 50, 100, 200, 300, 400, 500, 600, 800, 1000 or 1100, and a level of significance (α-error-level) of e.g. 0.05 or smaller, e.g. 0.05, preferably 0.01 or smaller. A statistical value can be obtained for each structural variation and/or each nucleic acid / genetic sequence in the genome as well as for all antibiotics tested, a group of antibiotics or a single antibiotic. The obtained p-values can also be adapted for statistical errors, if needed.

For statistically sound results a multitude of individuals should be sampled, with n = 50, 100, 200, 300, 400, 500, 600, 800, 1000, or 1100 and a level of significance (α-error-level) of e.g. 0.05 or smaller, e.g. 0.05, preferably 0.01 or smaller. According to certain embodiments, particularly significant results can be obtained for n = 200, 300, 400, 500, 600.

For statistically sound results a multitude of individuals should be sampled, with n = 50 or more, 100 or more, 200 or more, 300 or more, 400 or more, 500 or more, 600 or more, 800 or more, 1000 or more, or 1100 or more, and a level of significance (α-error-level) of e.g. 0.05 or smaller, e.g. 0.05, preferably 0.01 or smaller. According to certain embodiments, particularly significant results can be obtained for n = 200 or more, 300 or more, 400 or more, 500 or more, 600 or more, 800 or more, 1000 or more, or 1100 or more.

When referring to the second data set, wherein the second data set e.g. comprises, respectively is, a set of antimicrobial drug, e.g. antibiotic, resistances of a plurality of clinical isolates, this can, within the scope of the invention, also refer to a self-learning data base that, whenever a new sample is analyzed, can take this sample into the second data set and thus expand its data base. The second data set thus does not have to be static and can be expanded, either by external input or by incorporating new data due to self-learning. This is, however, not restricted to the first aspect of the invention, but applies to other aspects of the invention that refer to a second data set, which does not necessarily have to refer to antimicrobial drug resistance. The same applies, where applicable, to the first data set, e.g. in the first aspect.

According to certain embodiments of the first aspect, the structural variations are detected alignment-free. According to certain embodiments, the structural variations are annotated to a pan-genome of the microorganism and/or annotated to one or more reference sequences.

According to certain embodiments, statistical analysis in the present methods is carried can be carried using Fisher's test with p < 10⁻³, preferably p < 10⁻⁶, further preferably p < 10⁻⁹

The method of the first aspect of the present invention, as well as related methods, e.g. according to the 2^{nd} and 3^{rd} as-pect, can, according to certain embodiments, comprise correlating different genetic sites to each other. This way even higher statistical significance can be achieved.

According to certain embodiments of the method of the first aspect and related methods - as above, the second data set can be provided by culturing the clinical isolates of the microorganism on agar plates provided with antimicrobial drugs, e.g. antibiotics, at different concentrations, and the second data can be obtained by taking the minimal concentration of the plates that inhibits growth of the respective microorganism.

According to certain embodiments the antimicrobial drug, e.g. antibiotic drug, is selected from the group consisting of β-lactams, β-lactam inhibitors, quinolones and derivatives thereof, e.g. fluoroquinolones, aminoglycosides, glycopeptides, lincosamides, macrolides, nitrofuranes, oxazolidinones, polyketides, respectively tetracyclines, and folate synthesis inhibitors, e.g. benzene derived/sulfonamide antibiotics. According to certain embodiments, the antimicrobial drug, e.g. antibiotic drug, is selected from the group consisting of Amoxicillin/K Clavulanate (AUG), Ampicillin (AM), Aztreonam (AZT), Cefazolin (CFZ), Cefepime (CPE), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S). According to certain embodiments, the microorganism is a Gram-positive or a Gram-negative bacteria, e.g. a Gram-negative bacteria.

In the methods of the invention, the resistance of the microorganism, particularly the bacterial microorganism, to one or more antimicrobial, e.g. antibiotic, drugs can be determined.

According to certain embodiments, the resistance of a microorganism, particularly bacterial microorganism, against 1, 2, 3, 4, 5, 6, 7, 8, 9, 10, 11, 12, 13, 14, 15 or 16, 17, 18, 19, 20, 21 or more antibiotic drugs is determined. According to certain embodiments, the resistance of a microorganism, particularly bacterial microorganism, against 1, 2, 3, 4, 5, 6, 7, 8, 9, 10, 11, 12, 13, 14, 15 or 16, 17, 18, 19, 20 or 21 antibiotic drugs is determined.

A second aspect of the present invention relates to a diagnostic method of determining an infection of a patient with a microorganism, particularly a bacterial microorganism potentially resistant to antimicrobial drug treatment, comprising the steps of:
a) obtaining or providing a sample containing or suspected of containing a microorganism, particularly a bacterial microorganism, from the patient;
b) determining the presence of at least one structural variation of the genome comprising at least a change in the genome comprising more than one base, and at least one single nucleotide polymorphism (SNP) in at least one genetic sequence of the microorganism, particularly bacterial microorganism, as determined by the method of the first aspect, wherein the presence of said at least one structural variation and said single nucleotide polymorphism is indicative of an infection with an antimicrobial drug resistant microorganism in said patient.

An infection of a patient with a microorganism, preferably a bacterial microorganism, e.g. one or more of Acinetobacter, Escherichia, e.g. E.coli, Enterobacter, Klebsiella, Proteus, Pseudomonas, Salmonella, Serratia, Shigella and/or Staphylococcus species, potentially resistant to antimicrobial drug treatment herein means an infection of a patient with a microorganism, preferably a bacterial microorganism, particularly one as noted above, wherein it is unclear if the microorganism, preferably bacterial microorganism, is susceptible to treatment with a specific antimicrobial drug or if it is resistant to the antimicrobial drug.

With this method, any mutations in the genome of a microorganism, e.g. bacterial microorganism, e.g. a clinical isolate with an unknown strain of the microorganism, particularly bacterial microorganism, correlated with antimicrobial drug, e.g. antibiotic, resistance can be determined and a thorough antimicrobial drug, e.g. antibiotic, resistance profile can be established comprising structural variations as well as SNPs.

Again, the different steps can herein be carried out as described with regard to the first aspect of the present invention.

According to this aspect, an infection with a microorganism, particularly a bacterial microorganism, in a patient can be determined using sequencing methods, as well as a resistance to antimicrobial drugs, e.g. antibiotics, of the microorganism can be determined in a short amount of time compared to conventional methods, and a more thorough diagnostic is possible compared to a determination of only structural variations or SNPs, leading to improved results for determining the resistance and/or susceptibility of the microorganism, particularly bacterial microorganism.

In a third aspect, the present invention relates to a treatment of a patient suffering from an infection with a potentially resistant microorganism, particularly bacterial microorganism, comprising the steps of:
a) obtaining or providing a sample containing or suspected of containing a microorganism, particularly a bacterial microorganism, from the patient;
b) determining the presence of at least one structural variation of the genome comprising at least a change in the genome comprising more than one base, and at least one single nucleotide polymorphism (SNP) in at least one genetic sequence of the microorganism, particularly bacterial microorganism, as determined by the method of the first aspect, wherein the presence of said at least one structural variation is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism, particularly the bacterial microorganism.

This method can be carried out similarly to the second aspect of the invention and enables a fast way to select a suitable treatment with antibiotics for any infection with an unknown microorganism, particularly bacterial microorganism, with improved results compared to a determination of only structural variations or SNPs.

In this method, as well as similar ones, no aligning is necessary, as the unknown sample can be directly correlated, after the genome or genome sequences are produced, with the second data set, and thus genetic variations and antimicrobial drug, e.g. antibiotic, resistances can be determined. The first data set can be assembled, for example, using known techniques.

According to certain embodiments, statistical analysis in the present method is carried out using Fisher's test with p < 10⁻³, preferably p < 10⁻⁶, preferably p < 10⁻⁹. Also, according to certain embodiments, the method further comprises correlating different genetic sites to each other.

According to certain aspects, structural variations and/or SNPs in at least two, three, four, five, six, seven, eight, nine or ten positions, respectively sequences, are determined in any of the methods of the present invention, e.g. in at least two positions, respectively sequences, or in at least three positions, respectively sequences. Instead of testing only single positions, respectively sequences, the combination of several variant positions, respectively sequences, can improve the prediction accuracy and further reduce false positive findings that are influenced by other factors. Therefore, it is in particular preferred to determine the presence of structural variations and SNPs in 2, 3, 4, 5, 6, 7, 8 or 9 (or more) sequences.

The identification of the at least one or more antimicrobial, e.g. antibiotic, drug in step c) is then based on the results obtained in step b) and corresponds to the antimicrobial, e.g. antibiotic, drug(s) that correlate(s) with the structural variations and SNPs. Once these antimicrobial drugs, e.g. antibiotics, are ruled out, the remaining antimicrobial drugs, e.g. antibiotic drugs/antibiotics, can be selected in step d) as being suitable for treatment.

According to certain embodiments in the second or third aspect, step b) is carried out using a classification approach/method like a decision tree, random forest, neural network, bayesian classification, support vector machine, etc. wherein at first the presence of a single nucleotide polymorphism is determined, e.g. a decision tree, wherein in the decision tree at first the presence of a single nucleotide polymorphism is determined. A classification approach can be suitably selected and applied, e.g. a decision tree can be generated using known methods, e.g. within the scope of the statistical analysis, and is otherwise not particularly restricted. According to certain embodiments, a resistance in the microorganism can be determined using a decision tree, corresponding to a statistical analysis, wherein one or more SNPs are determined prior to determining one or more structural variants. This way the diagnosis of a resistant microorganism, e.g. bacterial microorganism, can be optimized.

According to certain embodiments, determining the nucleic acid sequence information or the presence of a genetic variation in the present methods comprises using a next generation sequencing or high throughput sequencing method, e.g. as mentioned above.

According to certain embodiments, the antibiotic is choses from Ampicillin-sulbactam (A/S) and Levofloxacin (LVX). According to certain embodiments, the microorganism, particularly bacterial microorganism, in the present methods is chosen from bacterial microorganisms from the genus Escherichia and/or Klebsiella, particularly *E. coli* and/or *K. pneumoniae.* According to certain embodiments, the antibiotic is chosen for Escherichia, particularly *E. coli,* from the group consisting of Amoxicillin-clavulanate (AUG), Ampicillin-sulbactam (A/S) and Levofloxacin (LVX), particularly Ampicillin-sulbactam (A/S) and Levofloxacin (LVX). According to certain embodiments, the antibiotic is chosen for Klebsiella, particularly *K. pneumoniae,* from the group consisting of Ampicillin-sulbactam (A/S) and Levofloxacin (LVX).

A fourth aspect of the present invention relates to a method of determining structural variations and SNPs of a genome of a microorganism for a clinical isolate of the microorganism, particularly a bacterial microorganism, comprising: obtaining or providing at least one nucleic acid, e.g. gene, sequence of the clinical isolate of the microorganism, particularly the bacterial microorganism; and
determining the presence of structural variations and SNPs in the at least one nucleic acid, e.g. gene, sequence of the clinical isolate of the microorganism, particularly bacterial microorganism, as determined by the method of the first aspect.

With this method, antimicrobial drug, e.g. antibiotic, resistances in an unknown isolate of a microorganism, e.g. bacterial microorganism, can be determined.

A simple read out concept for a diagnostic test as described in this aspect can be as follows.

A sample, e.g. blood from a patient, is used for molecular testing, e.g. using next generation sequencing (NGS), and then a molecular fingerprint is taken, e.g. in case of NGS a sequence of selected genomic/plasmid regions or the whole genome is assembled. This is then compared to a reference library containing several reference sequences and/or a pan-genome, i.e. selected sequences or the whole sequence are/is compared to one or more reference sequences and/or a pan-genome, and structural variations (sequence / gene additions/deletions, etc.) and SNPs are correlated with susceptibility/resistance profiles of reference sequences of the reference library. The reference library herein contains many genomes and/or one or more pan-genomes and is different from a reference genome. Then the result is reported, which can comprise ID (pathogen identification), i.e. a list of all (pathogenic) species identified in the sample, and AST (antimicrobial susceptibility testing), i.e. a list including a susceptibility /resistance profile for all species listed, based on structural variations.

According to certain embodiments, statistical analysis in the present method is carried out using Fisher's test with p < 10⁻³, preferably p < 10⁻⁶, preferably p < 10⁻⁹. Also, according to certain embodiments, the method further comprises correlating different genetic sites to each other.

Again, in the second, third and fourth aspect, the different steps herein can be carried out as described with regard to the first aspect of the present invention.

According to certain embodiments, the obtaining or providing of a sample containing or suspected of containing at least one microorganism, preferably a bacterial microorganism, e.g. one or more of Acinetobacter, Escherichia, e.g. E.coli, Enterobacter, Klebsiella, Proteus, Pseudomonas, Salmonella, Serratia, Shigella and/or Staphylococcus species, from the patient in the methods of the invention can comprise the following:
A sample of a vertebrate, e.g. a human, e.g. is provided or obtained and nucleic acid sequences, e.g. DNA or RNA sequences, are recorded by a known method for recording nucleic acid, which is not particularly limited. For example, nucleic acid can be recorded by a sequencing method, wherein any sequencing method is appropriate, particularly sequencing methods wherein a multitude of sample components, as e.g. in a blood sample, can be analyzed for nucleic acids and/or nucleic acid fragments and/or parts thereof contained therein in a short period of time, including the nucleic acids and/or nucleic acid fragments and/or parts thereof of the microorganism. For example, sequencing can be carried out using polymerase chain reaction (PCR), particularly multiplex PCR, or high throughput sequencing or next generation sequencing, preferably using high-throughput sequencing. For sequencing, preferably an *in vitro* sample is used.

The data obtained by the sequencing can be in any format, and can then be analyzed as described with regard to the first to fourth aspect of the present invention.

In a fifth aspect the present invention relates to one or more computer program products comprising computer executable instructions which, when executed, perform a method according to any one of the first to the fourth aspect of the present invention.

In certain embodiments the computer program product is one on which program commands or program codes of a computer program for executing said method are stored. According to certain embodiments the computer program product is a storage medium. As noted above, the computer program products of the present invention can be self-learning, e.g. with respect to the first and second data sets.

In order to obtain the best possible information from the highly complex genetic data and develop an optimum model for diagnostic and therapeutical uses as well as the methods of the present invention - which can be applied stably in clinical routine - a thorough in silico analysis can be necessary. The proposed principle is based on a combination of different approaches, e.g. assembly of the nucleic acid, e.g. gene, sequences and/or genome of the microorganisms, at least in part and optionally annotating the sequences to one or more reference sequences and/or one or more pan-genomes, and/or alignment of the sequence data of the clinical isolate to be determined with one or more reference sequences and/or one or more pan-genomes, and correlation of structural variations and SNPs found in every sample, e.g. from each patient, respectively an unknown clinical isolate, with all references and drugs, e.g. antibiotics, or only one or some of them, and search for structural variations and SNPs which occur in one or several drugs and one or several strains.

Using the above steps a list of structural variations and SNPs with regard to one or more reference sequences and/or one or more pan-genomes is generated. These can be stored in databases and statistical models can be derived from the databases. The statistical models can be based on at least one or more structural variations and at least one or more SNPs in at least one or more sequences. Statistical models that can be trained can be combined from structural variations, SNPs and sequences. Examples of algorithms that can produce such models are association Rules, Support Vector Machines, Decision Trees, Decision Forests, Discriminant-Analysis, Cluster-Methods, and many more.

The goal of the training is to allow a reproducible, standardized application during routine procedures.

For this, for example, nucleic acid, e.g. gene, sequences or parts thereof can be sequenced from a patient to be diagnosed. Afterwards, core characteristics can be derived from the sequence data which can be used to predict resistance. These are the points in the database used for the final model, i.e. at least one structural variation and one SNP, but also combinations of one or more structural variations and one or more SNPs, etc.

The corresponding characteristics can be used as input for the statistical model and thus enable a prognosis for new patients. Not only the information regarding all resistances of all microorganisms, against all or only some or one drugs, e.g. antibiotics, can be integrated in a computer decision support tool, but also corresponding directives (e.g. EUCAST) so that only treatment proposals are made that are in line with the directives.

A sixth aspect of the present invention relates to the use of the computer program product according to the fifth aspect, e.g. for determining structural variations and SNPs of a genome of a microorganism for a clinical isolate of the microorganism in the fourth aspect of the invention and/or for use in the diagnostic method of the second method of the invention and/or for selecting a treatment in the third aspect of the present invention and/or in the method of the first aspect of the present invention.

A seventh aspect of the present invention is directed to a method of treating a patient suffering from an antimicrobial drug, e.g. antibiotic, resistant infection with a microorganism, preferably a bacterial microorganism, e.g. one or more of Acinetobacter, Escherichia, e.g. E.coli, Enterobacter, Klebsiella, Proteus, Pseudomonas, Salmonella, Serratia, Shigella and/or Staphylococcus species, comprising the steps of:
a) obtaining or providing a sample containing or suspected of containing at least one microorganism, preferably a bacterial microorganism, from the patient;
b) determining the presence of at least one structural variation of the genome comprising at least a change in the genome comprising more than one base, and at least one single nucleotide polymorphism (SNP) in at least one genetic sequence of the microorganism, particularly bacterial microorganism, as determined by the method of the first aspect, wherein the presence of said at least one structural variation is indicative of a resistance to one or more antimicrobial drugs;
c) identifying said at least one or more antimicrobial, e.g. antibiotic, drugs;
d) selecting one or more antimicrobial, e.g. antibiotic, drugs different from the ones identified in step c) and being suitable for the treatment of the infection with the microorganism, preferably a bacterial microorganism; and
e) treating the patient with said one or more antimicrobial, e.g. antibiotic, drugs.

Herein, steps a) to d) can be carried out as described with respect to the fourth aspect. Step e) can be sufficiently carried out without being restricted and can be done e.g. non-invasively.

### Examples

The present invention will now be described in detail with reference to several examples thereof. However, these examples are illustrative and do not limit the scope of the invention.

In the following Example, two different microorganisms, namely *Escherichia coli* and *Klebsiella pneumoniae,* were tested for identification of antibiotic resistances using structural variations and SNPs separately, respectively, as well as tested for identification of antibiotic resistances using a combined approach using structural variations and SNPs.

In the Example, the consideration of structural variations in the genomes is also referred to as "approach A", and the consideration of single nucleotide variations, i.e. SNPs, is also referred to as "approach B".

To test both approaches, we evaluated pathogenic *E. coli* and *K. pneunomiae* bacteria as proof-of-concept. Specifically, we generated genetic profiles for 1,161 pathogenic *E. coli* and 1,171 *K. pneumoniae* isolates by using whole genome deep sequencing. For the isolates, we performed standard culturebased resistance tests for 21 different drugs, Amoxicillin/K Clavulanate (AUG), Ampicillin (AM), Aztreonam (AZT), Cefazolin (CFZ), Cefepime (CPE), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T, or P_T), Ampicillin/Sulbactam (A/S, or A_S), Tetracycline (TE), Tobramycin (TO), and Trimethoprim/Sulfamethoxazole (T/S, or T_S). These drugs belong to five different drug classes, i.e. β-lactam antibiotics, quinolone antibiotics, aminoglycoside antibiotics, polyketide antibiotics, and benzene derived/sulfonamide antibiotics. For both approaches computer-aided analysis and machine learning technologies have been employed.

For approach A the following procedure has been carried out: First, a pan genome for *E. coli* and *K. pneumoniae* was defined respectively using the database MetaRef. Lists of centroids (gene cluster representatives) for *E. coli* (20,639 unique centroids) and *K. pneumoniae* (5,860 unique centroids) were used and their nucleotide sequences were extracted. Then, the centroids were aligned against the de novo assemblies of the 1,161 and 1,171 bacteria samples using BLASTn with the following parameters: word size = 11, gap open = 3, gap extend = 2, penalty = -2, reward = 1. For each alignment result the matches were sorted by centroid ID, centroid alignment length, identity and E-value to extract best hits for each centroid and each isolate. A centroid was considered as present in an isolate if its best hit had at least 80% identity and at least 80% of the centroid sequence length was aligned. In that we generated a structural information map, defining for each sample, which nucleic acid sequences, e.g. genes, on the core and pan genome were present or not in the respective samples. These binary matrices (one for *E. coli* and one for *K. pneunomiae)* were subjected to different statistical learning approaches. In this analysis the maximum tree depth was set to 10, no pruning was performed, no surrogate splits were used, and the complexity parameter was set to 0.01, the minimal split number was set to 2. To account for class imbalance a loss matrix was used where the penalty for predicting resistance instead of susceptibility was defined as the ratio of resistant samples in the data set (the penalty for predicting susceptibility instead of resistant was defined analogously). To test for potential over-training of the models non-parametric permutation tests were done.

For approach B, 40 representative samples of *E. coli* and of *K. pneumoniae* were randomly selected and matched against all currently available reference genomes. In the analysis no substantial variations between the reference genomes were observed. The best of all reference genomes was then used to align all *E. coli* and *K. pneunomiae* samples against it (Klebsiella pneumoniae subsp. pneumoniae MGH 78578; Escherichia coli str. K-12 substr. DH10B chromosome). From these alignments, single nucleotide variants (SNPs) were calculated and the respective matrices containing the genetic variations were subjected to the same statistical learning approach as for approach A.

The results of both approaches are presented in detail in Tables 1 and 2. The tables present the mean accuracy, sensitivity, specificity, PPV (positive predictive value) and ROC curve (receiver operating characteristic) AUC (area under curve) value. The upper numbers in each table denote the results from approach B and the lower numbers in each column of approach A for the respective antimicrobial drug. The bold, underlined numbers highlight the better performing approach. The average accuracy of approach A was for *E. coli* 80% and for approach B 87%. For *K. pneunomiae,* approach A had an average performance of 87% and approach B of 87.3%.

**Table 1: Test results for E. coli, with the upper line for each antibiotic representing approach B (SNPs), and the lower line representing approach A (structural variations)**

| Drug | Abbr. | Accuracy | Sensitivity | Specificity | PPV | AUC |
|---|---|---|---|---|---|---|
| Ampicillin-sulbactam | A_S | **90** | **93** | **90** | **90.2** | **92.9** |
| | | 67 | 68 | 65.8 | 68 | 70.5 |
| Ampicillin | AM | **93.9** | **92.6** | **95.3** | **95.8** | **96.1** |
| | | 69.4 | 70.4 | 68.3 | 73 | 73.3 |
| Amoxicillin-clavulanate | AUG | **74.3** | **82.2** | **70.8** | **56.7** | **79.5** |
| | | 66.1 | 59.2 | 69.4 | 49 | 65 |
| Aztreonam | AZT | **89.6** | **80** | **90. 6** | **47.2** | **86.3** |
| | | 85.3 | 60.8 | 87.8 | 34.3 | 72 |
| Ceftriaxone | CAX | **89.4** | **76.9** | **90. 9** | **49.5** | **82** |
| | | 83.1 | 57 | 86.1 | 32.4 | 72.9 |
| Cefotaxime | CFT | **89.1** | **76.6** | **90.6** | **51** | **84.2** |
| | | 83.7 | 58 | 86.8 | 34.9 | 72.5 |
| Ciprofloxacin | CP | 89.6 | 82.9 | 91.1 | 68.1 | 87.9 |
| | | **97.7** | **96.1** | **98.1** | **92.5** | **97.4** |
| Cefuroxime | CRM | **79.6** | **67.7** | **82.3** | **47.4** | **79** |
| | | 73.8 | 59 | 77.1 | 37.8 | 68 |
| Gentamicin | GM | 82.4 | **77.2** | 83.1 | **37.5** | **77.6** |
| | | 83. 9 | 50 | 88. 6 | 35.2 | 65.1 |
| Levofloxacin | LVX | 90 | 84.6 | 92.2 | 71.1 | 89.4 |
| | | **97** | **96.6** | **98** | **91.9** | **97.4** |
| Trimethoprimsulfamethoxazole | T_S | 90 | 92.9 | 88.8 | 77.9 | 92 |
| | | 68.6 | 61.9 | 71.6 | 48.9 | 70 |
| Tobramycin | TO | 83.2 | **74.9** | 84.2 | 35.4 | **76.2** |
| | | **83.9** | 66.5 | **86** | **36.5** | 74.9 |

| | | | | | | |
|---|---|---|---|---|---|---|
| Abbr. = abbreviation; PPV = positive predictive value; AUC = area under curve | | | | | | |

**Table 2: Test results for K. pneumoniae, with the upper line for each antibiotic representing approach B (SNPs), and the lower line representing approach A (structural variations)**

| Drug | Abbr. | Accuracy | Sensitivity | Specificity | PPV | AUC |
|---|---|---|---|---|---|---|
| Ampicillin-sulbactam | A_S | **80** | **73.1** | **88.7** | **86.6** | **83.6** |
| | | 76 | 68.9 | 82.9 | 79.9 | 79.6 |
| Amoxicillin-clavulanate | AUG | **86.6** | **85.9** | 86.9 | **72.8** | **89.3** |
| | | 83.3 | 74.6 | **87.1** | 70.9 | 82.1 |
| Aztreonam | AZT | 89.4 | **89.2** | 89.6 | 76.7 | **92.5** |
| | | **91.6** | 86.9 | **93.4** | **84** | 91.4 |
| Ceftriaxone | CAX | 89.5 | **90.3** | 89.2 | 77.3 | **92.4** |
| | | **90.9** | 86.2 | **92.8** | **82.9** | 90.9 |
| Ceftazidime | CAZ | 89.3 | **87.9** | 89.9 | 78.1 | **92.5** |
| | | **90.9** | 85.4 | **93.2** | **84.2** | 89.9 |
| Cefotaxime | CFT | 90.2 | **91.1** | 89.8 | 77.6 | **92.3** |
| | | **91.3** | 84.8 | **93.7** | **83.9** | 89.9 |
| Ciprofloxacin | CP | 85.6 | 77.8 | 88.1 | 68.2 | 84.3 |
| | | **91.9** | **87.3** | **93.4** | **81.3** | **91.2** |
| Cefepime | CPE | 88.1 | **83.3** | 89.1 | 60.9 | **86.5** |
| | | **88.3** | 81.2 | **89.7** | **62.6** | 86.1 |
| Cefuroxime | CRM | **82.9** | 74.4 | **87.9** | **78.8** | 83 |
| | | 81.8 | **74.7** | 86 | 76.8 | **83.7** |
| Ertapenem | ETP | 94.1 | **84.8** | 95.2 | 67.5 | 88.3 |
| | | **94.7** | 82.3 | **96.3** | **73.6** | **88.8** |
| Gentamicin | GM | **83.6** | **88.2** | 82.6 | **53.6** | **86.8** |
| | | 79.8 | 55.5 | **85.4** | 46.1 | 70.3 |
| Levofloxacin | LVX | 84.8 | 77.7 | 86.9 | 62.9 | 85.1 |
| | | **90** | **86.7** | **92.9** | **78.2** | **91.3** |
| Piperacillintazobactam | P_T | 83.7 | **83.2** | 83.8 | 62.9 | **86.1** |
| | | **85.1** | 76.9 | **87.8** | **67.9** | 83.3 |
| Trimethoprimsulfamethoxazole | T_S | **88.5** | **94.8** | 86.1 | **71.1** | **92.2** |
| | | 80.4 | 72.7 | 83.2 | 61.6 | 79.7 |
| Tobramycin | TO | **91.6** | **91.8** | **91.5** | **79.6** | **95.5** |
| | | 88.1 | 79.5 | 91.2 | 77.4 | 87.5 |

We observed highly variable results that indicated that in certain cases approach A and in other cases approach B were more accurate. This was even not concordant between *E. coli* and *K. pneunomiae.* Since in many indications the resistance / sensitivity for a broad number of drugs is required, we concluded that a combination of approach A and B, i.e. combining the structural genomic information on structural variations, e.g. gene presence and absence, combined with the genetic variants on single nucleotide level, i.e. SNPs, has a substantially higher performance and enables accurate genetic resistance testing.

For *E. coli* a combined decision tree analysis according to the one in approach A and B (initial performance 80% and 87%) increased the accuracy to 88.4% (+1.4%). Likewise, the performance for *K. pneunomiae* was increased by 1.4% from 87.3% to 88.7%. Further, extreme cases were observed. For *E. coli* the minimal performance of the single approaches was 66.1% for Amoxicillin-clavulanate (AUG). For the combined approach the performance jumped by 8.2% to 74.3%. Likewise, for *K. pneunomiae* the performance increased for Ampicillin-sulbactam (A_S) from 76% to 80.9%. In addition, the following remarkable results of Table 3 were achieved.

**Table 3: Selected results from combined approach**

| **Organism** | **Drug** | **Accuracy SNPs** | **Accuracy Structural Variations** | **Accuracy Combined Model** |
|---|---|---|---|---|
| *E. coli* | Ampicillin-sulbactam (A/S) | 67 | 90 | 95 |
| *E. coli* | Levofloxacin (LVX) | 97 | 90 | 99 |
| *K. pneumoniae* | Ampicillin-sulbactam (A/S) | 76 | 80 | 90 |
| *K. pneumoniae* | Levofloxacin (LVX) | 90 | 85 | 95 |

These numbers impressively demonstrate on a large data set that the combination of single nucleotide level information with larger genetic variations has a substantial potential to improve diagnosis, prognosis and therapy stratification in human pathology.

## Claims

1. A method of determining an antimicrobial drug resistance profile for a microorganism, comprising:
obtaining or providing a first data set of nucleic acid sequences of a plurality of clinical isolates of the microorganism, wherein optionally at least a part of the nucleic acid sequences of the first data set are assembled; and/or obtaining or providing a first data set of nucleic acid sequences of a plurality of clinical isolates of the microorganism and aligning the nucleic acid sequences of the first data set to at least one reference sequence;
analyzing the nucleic acid sequences of the first data set for structural variations of the genome comprising at least a change in the genome comprising more than one base, and analyzing the nucleic acid sequences of the first data set for single nucleotide polymorphisms (SNPs) to obtain a third data set of structural variants;
providing a second data set of antimicrobial drug, e.g. antibiotic, resistance and/or susceptibility of the plurality of clinical isolates of the microorganism;
correlating the third data set with the second data set and statistically analyzing the correlation; and
determining the structural variations and SNPs in the genome of the microorganism associated with antimicrobial drug, e.g. antibiotic, resistance.

2. The method of claim 1, wherein the structural variations and the SNPs are annotated to a pan-genome of the microorganism and/or annotated to one or more reference genomes.

3. The method of one or more of the preceding claims, wherein the method involves determining the resistance of the microorganism to one or more antimicrobial drugs.

4. The method of one or more of the preceding claims, wherein the antimicrobial drug is selected from the group consisting of Amoxicillin/K Clavulanate (AUG), Ampicillin (AM), Aztreonam (AZT), Cefazolin (CFZ), Cefepime (CPE), Cefotaxime (CFT), Ceftazidime (CAZ), Ceftriaxone (CAX), Cefuroxime (CRM), Cephalotin (CF), Ciprofloxacin (CP), Ertapenem (ETP), Gentamicin (GM), Imipenem (IMP), Levofloxacin (LVX), Meropenem (MER), Piperacillin/Tazobactam (P/T), Ampicillin/Sulbactam (A/S), Tetracycline (TE), Tobramycin (TO), and Trimethoprim/Sulfamethoxazole (T/S).

5. The method of one or more of the preceding claims, wherein the resistance of the microorganism against 1, 2, 3, 4, 5, 6, 7, 8, 9, 10, 11, 12, 13, 14, 15 or 16, 17, 18, 19, 20 or 21 antibiotic drugs is determined.

6. A method of determining an infection of a patient with a microorganism, comprising the steps of:
a) obtaining or providing a sample containing or suspected of containing a microorganism from the patient;
b) determining the presence of at least one structural variation of the genome comprising at least a change in the genome comprising more than one base, and at least one single nucleotide polymorphism (SNP) in at least one genetic sequence of the microorganism, as determined by the method of any one of claims 1 to 5, wherein the presence of said at least one structural variation and said at least one single nucleotide polymorphism is indicative of an infection with an antimicrobial drug resistant microorganism in said patient.

7. The method of claim 6, wherein the microorganism is a bacterial microorganism potentially resistant to antimicrobial drug treatment.

8. A method of selecting a treatment of a patient suffering from an infection with a potentially resistant microorganism, comprising the steps of:
a) obtaining or providing a sample containing or suspected of containing a microorganism from the patient;
b) determining the presence of at least one structural variation of the genome comprising at least a change in the genome comprising more than one base, and at least one single nucleotide polymorphism (SNP) in at least one genetic sequence of the microorganism, as determined by the method of any one of claims 1 to 5, wherein the presence of said at least one structural variation and said at least one single nucleotide polymorphism is indicative of a resistance to one or more antimicrobial drugs;
c) identifying said at least one or more antimicrobial drugs; and
d) selecting one or more antimicrobial drugs different from the ones identified in step c) and being suitable for the treatment of the infection with the microorganism.

9. The method of one or more of claims 6 to 8, wherein step b) is carried out using a classification approach, wherein at first the presence of a single nucleotide polymorphism is determined.

10. The method of one or more of claims 6 to 9, wherein determining the nucleic acid sequence information or the presence of a genetic variation comprises using a next generation sequencing or high throughput sequencing method.

11. The method of one or more of the preceding claims, wherein the microorganism is chosen from bacterial microorganisms from the genus Escherichia and/or Klebsiella.

12. Computer program product comprising computer executable instructions which, when executed, perform a method according to any one of claims 1 to 11.
